# EUROPÄISCHE PATENTSCHRIFT

(11) **EP 0 505 763 B1**
(45) Veröffentlichungstag und Bekanntmachung des Hinweises auf die Patenterteilung: **06.11.1996**
(21) Anmeldenummer: 92103435.1
(22) Anmeldetag: 28.02.1992
(51) Int. Cl.: A01N 37/36, A61L 2/18

(54) **Wässriges Desinfektionsmittel, seine Verwendung zur Inaktivierung von Hepatitis-B-Viren, bakteriellen Sporen und Legionella pneumophila sowie Desinfektionsverfahren**
Aqueous disinfectant, its use for inactivation of Hepatitus-B virus, bacterial spores and Legionella pneumophila, and method of disinfection
Désinfectant aqueux, son utilisation pour l'inactivation des virus Hepatitus-B, spores bactériennes et Legionella pneumophila, et procédé pour la désinfection

(30) Priorität: 27.03.1991 DE 4110078; 06.01.1992 DE 4200066
(43) Veröffentlichungstag der Anmeldung: 30.09.1992
(73) Patentinhaber: Fresenius AG, D-61350 Bad Homburg (DE)
(72) Erfinder: Kirschner, Ulrich, Dr., W-6082 Mörfelden-Walldorf (DE); Pohl, Thomas, W-6380 Bad Homburg v.d.H. 6 (DE)
(74) Vertreter: Fuchs, Luderschmidt & Partner Patentanwälte

(56) Entgegenhaltungen:
- EP-A- 0 148 709
- EP-A- 0 251 303
- AT-B- 382 310
- AU-B- 64 117
- GB-A- 2 103 089
- BIOLOGICAL ABSTRACTS, Band 56, 1973, Philadelphia, PA (US; J. WISNIEWSKI, AN 1433
- FOOD CHEMISTRY, Band 4, Nr. 4, Oktober 1979, Essex, England (GB); G. POLI et al., Seiten 251-258
- AMERICAN JOURNAL OF HYGIENE, Band 16, 1932; J.D. REID, Seiten 540-556

## Beschreibung

Die Erfindung betrifft die Verwendung eines wäßrigen Desinfektionsmittels bei der Desinfektion insbesondere thermolabiler medizinischer Instrumente, Geräte, Dialysemaschinen sowie Teile derselben und Flächen.

Die Verwendung von zitronesäurehaltigen Desinfektionsmitteln gehört bereits zum Stand der Technik.

So offenbart die GB-2 103 089 die Verwendung von Carbonsäuren als Viruzide. Beschrieben wird die Dekontamination von Parainfluenzaviren (eingehüllte Viren) mit Carbonsäuren wie Zitronersäure, Äpfelsäure, Bernsteinsäure und Benzoesäure, von Rhinoviren (nackte Viren) mit Carbonsäuren zusammen mit einer Trägersubstanz (z.B. einem Kleenextuch/und Adenovirus (nackter Virus) mit Carbonsäuren, einem Träger und einem Tensid.

Die AT-382 310 betrifft die Entkalkung, Desinfektion und Reinigung von Hygienegeräten wie Zahnbürsten etc. Das für die Desinfektion zu verwendende Gemisch besteht aus 15-80 Gew.-%, insbesondere 15-30 Gew.-%, Milch-, Glycol-, Wein-, Zitronen-, Ameisen, Essig-, and/oder Proprionsäure, 10-60 Gew.-%, insbesondere 10-40 Gew.-% Ethanol, Isopropanol und/oder n-Propanol und mindestens 5 Gew.-%, insbesondere 20-60 Gew.-% Wasser, sowie Spuren von etherischen Ölen. Das bekämpfte Keimspektrum umfaßt neben Pseudomonaden Staphylokokken, Enterobakterien, aerobe Sporenbildner, Sproßpilze und Nonfermenter. Es ist klar, daß die AT-382 310 keine wäßrigen sondern alkoholische Zubereitungen offenbart.

Auch die EP-0 251 303 offenbart viruzide Mittel mit Breitbandwirkung, die neben 0,5 bis 5 Gew.-% einer kurzkettigen organischen Säure mindestens 70 Gew.-% Ethanol und/oder Proponal enthalten. Damit handelt es sich um alkoholische Desinfektionsmittel deren gute Wirksamkeit zwar bekannt ist, die aufgrund ihres hohen Anteils an alkoholischen Substanzen jedoch nicht unbedenklich für die Umwelt sind. Es besteht damit ein Bedarf an wäßrigen Desinfektionsmitteln gerade im klinischen Bereich.

Eine Aufgabe der Erfindung besteht darin, der Desinfektion mit wäßrigen Desinfektionsmitteln neue klinische Anwendungsbereiche zu erschließen, wobei auf dem neuen Verwendungsgebiet eine hohe Wirksamkeit bei möglichst geringer Einwirkungszeit und möglichst niedrigen Einwirkungstemperaturen gewährleistet sein soll. Insbesondere in klinischen Bereichen, in denen die Verwendung von wäßrigen Desinfektionsmitteln bislang nicht angezeigt war, und das Risiko einer Infektion zu groß erschien, soll die Wirksamkeit gut sein.

Bekanntlich stellt gerade im Dialysebereich die Gefahr einer Hepatitis-Infektion ein ernstes Problem dar.

Es ist bekannt, daß zur rein thermischen Inaktivierung von Hepatitis-B-Viren eine Temperatur von 100°C bei einer Erhitzungsdauer von 10 Minuten oder aber eine Erhitzungsdauer von 10 Stunden auf eine Temperatur von 60°C, und von Hepatitis-A-Viren ein einminütiges Erhitzen auf 98°C erforderlich ist (vgl. K. H. Wallhäußer, "Praxis der Sterilisation, Desinfektion, Konservierung", 4. Aufl., 1988, Georg Thieme Verlag Stuttgart/New York, Seiten 75 ff. (Tabelle 31)). Desweiteren ist bekannt, daß Bakterien schon bei Temperaturen ab 60°C inaktiviert werden. Infolge der erforderlichen verhältnismäßig hohen Temperaturen bzw. langen Einwirkungsdauer ist diese Desinfektionsmethode insbesondere für hitzelabile Geräte aus Kunststoffen, wie zum Beispiel Dialysegeräten, unbrauchbar, weshalb derzeit eine Desinfektion von Geräten und Instrumenten durch Einlegen derselben für mindestens 6 Stunden in eine 3 %ige Formalinlösung erfolgt, wonach zwecks Vermeidung einer völlig unerwünschten Wirkstoffadsorption an den Kunststoffoberflächen sorgfältig gespült werden muß (vgl. K. H. Wallhäußer, a.a.O., S. 76). Abgesehen von der Umweltfeindlichkeit und Toxizität des Wirkstoffs Formaldehyd, sind derartige Verweilzeiten in der Praxis wenig akzeptabel.

Es wurde nun gefunden, daß die Aktivität des Hepatitis-B-Virus durch eine nur 1,5 (0,4) Gew.- %ige wäßrige Zitronensäurelösung bereits bei einer Einwirkungstemperatur von 20°C (60°C) und einer Einwirkungszeit von nur 5 Minuten auf eine virale Aktivität herabgesetzt werden kann, die im DNS-Polymerasetest nicht mehr meßbar war. Dies ist überraschend, nachdem nachgewiesen worden war, daß das bovine Parvovirus (BPV, Stamm Haden), das gegenüber chemischen Desinfektionsmitteln als sehr widerstandsfähig gilt, bei einer Temperatur von 30°C und weniger, selbst bei einer Einwirkungszeit von 6 Stunden durch Essig-, Propion-, Zitronen- oder sogar Ameisensäure, die sich bei höheren Temperaturen am wirksamsten erwies, in einer Konzentration von 3 % nicht bzw. nicht nennenswert inaktiviert wird (vgl. Hyg + Med 15 (1990), S. 313-317), und andererseits eine Inaktivierung des Hepatitis-A und Hepatitis-B-Virus durch Einstellung eines sauren pH-Bereichs auszuschließen war (vgl. K. H. Wallhäußer, a.a.O., Seiten 79/80).

Gegenstand der Erfindung ist nun die Verwendung eines gegebenfalls in Konzentration vorliegenden wäßrigen Desinfektionsmittels mit einem Gehalt an Zitronensäure als viruzidem Wirkstoff zur Inaktivierung von Hepatitis-B-Viren mit bakteriellen Sporen bei der Desinfektion von thermolabilen medizinischen Instrumenten und Geräten sowie Teilen derselben, Dialysegeräte und Flächen.

Das neue verwendete Desinfektionsmittel zeichnet sich gegenüber den bislang verwendeten, in aller Regel Formaldehyd enthaltend Desinfektionsmitteln durch die Umweltfreundlichkeit seiner viruziden Wirkstoffe aus; vor allem aber ermöglicht es wesentlich geringere Einwirkungszeiten, und dies bei ebenfalls relativ niedrigen Temperaturen.

So gelingt es z. B. mit Hepatitis-B-Viren kontaminierten Flächen, insbesondere von thermolabilen medizinischen Instrumenten und Geräten sowie Teilen derselben zu dekontaminieren indem man die Flächen bei einer Temperatur von etwa 20°C bis etwa 75°C während zumindest 1 bis 5 Minuten mit einer wäßrigen Lösung in Berührung bringt, die Zitronensäure in einer viruziden Konzentration enthält.

Eine besondere Ausführungsform des erfindungsgemäßen Desinfektionsmittels enthält Zitronensäure als viruziden Wirkstoff, vorzugsweise in einer Konzentration von 0,05 - 3 Gew.-%, vorzugsweise 0,2 - 2,5 Gew.-%, insbesondere etwa 0,4 - 0,6 Gew.-%.

Da die desinfizierende Wirkung mit der Erhöhung der Temperatur und Einwirkzeit zunimmt, ist die obere Grenze der Zitronensäurekonzentration speziell kurzen Einwirkzeiten, beispielsweise 5 - 10 Min., und niedrigen Temperaturen, beispielsweise die Raumtemperatur, zuzuordnen, während die unteren Grenzwerte entsprechend höheren Temperaturen eher 60°C und mehr sowie längeren Einwirkzeiten (15 Min. und mehr) zuzuordnen sind.

Die Wirkung der Zitronensäure kann durch Zugabe weiterer Säuren, insbesondere Äpfelsäure, Milchsäure und/oder Weinsäure verbessert werden. Zusätzlich können hierdurch Resistenzentwicklungen verhindert werden. Darüber hinaus kann die Neigung zur Kristallisation unterdrückt werden. Diese Säuren werden jeweils in einer Menge von 5 - 20 Gew.-%, vorzugsweise etwa 10 Gew.-%, des Zitronensäuregehalts eingesetzt.

Das erfindungsgemäße Desinfektionsmittel wird zweckmäßigerweise in Form eines Säurekonzentrats zur Verfügung gestellt, wobei sich eine besondere Ausführungsform durch einen Gehalt von 50 Gew.-% Zitronensäure auszeichnet. Das Konzentrat wird am Ort des Einsatzes mit sterilem, entionisiertem Wasser auf die niedrigere Konzentration, z.B. auf 2 Gew.-%, verdünnt.

Eine weitere besondere Ausführungsform in Form eines Konzentrats zeichnet sich durch einen Gehalt von 21 Gew.-% Zitronensäure, 2 Gew.-% Milchsäure und 2 Gew.-% Äpfelsäure aus.

Wie nachgewiesen wurde (vgl. nachfolgendes Beispiel) ermöglicht schon eine 1,5 Gew.-%ige wäßrige Zitronensäurelösung die völlige Inaktivierung von Hepatitis-B-Viren bei einer Einwirkungszeit von nur 5 Minuten und einer Einwirkungstemperatur von lediglich 20°C. Demgemäß kann man bei der Desinfektion unter erfindungsgemäßer Verwendung des Desinfektionsmittels die kontaminierten Flächen mit einer zumindest 1,5 Gew.-%igen, vorzugsweise 1,5 Gew.-%igen, wäßrigen Zitronensäurelösung bei einer Einwirkungstemperatur von 20 - 75°C, insbesondere 20°C, und während einer Einwirkungszeit von zumindest 5 Minuten, insbesondere 5 Minuten, in Berührung bringt.

Ein weiterer Gegenstand der Erfindung ist die Verwendung des wäßrigen zitronensäurehaltigen Desinfektionsmittels zur Inaktivierung von bakteriellen Sporen.

Nachfolgende Beispiele dienen zur näheren Erläuterung der Erfindung.

Soweit nicht anders angegeben, beziehen sich Prozentangaben auf das Gewicht.

### Beispiel

A Testmethode:
   Die hepatoviruziden Eigenschaften einer 2 Gew.-%igen Zitronensäurelösung wurden im Suspensionsversuch bei 20°C und bei 75°C untersucht.
   Da in vitro-Vermehrungssysteme für das Hepatitis-B-Virus (HBV) bislang noch nicht zur Verfügung stehen, wurde die Bestimmung der HBV-Infektiosität bei vorliegenden Versuchen mit dem DNS-Polymerase- Test vorgenommen. Die DNS-Polymerase stellt einen guten Marker für die strukturelle Integrität des Virus dar und kann deshalb als indirekter Marker für die Infektiositäten herangezogen werden.
C Zubereitung der Dane-Partikel-Suspension:
   Zur Konzentrierung und partiellen Reinigung der eingesetzten Dane-Partikel wurden 240 ml Serum von einem Probanden mit chronischem HBsAG-Trägerstatus (HBsAg = Hepatitis-B-surface -Antigen) und Nachweis von HBcAg (HBcAg = Hepatitis-B-core - Antigen) und der DNS-Polymerase in einer Zentrifuge vom Typ SW 28 Rotor bei 25.000 UpM während 16 Stunden bei 4°C zentrifugiert. Das Sediment wurde in 2 ml 0,01 M PBS mit 0,1 % (Gew./Vol.) Serumalbumin aufgenommen, und jeweils ein Milliliter wurde mit 4 ml einer 20 %igen (Gew./Gew.) Saccharose-Lösung unterschichtet.
   Eine Zentrifugation in einer Zentrifuge vom Typ SW 60 Ti Rotor bei 50.000 UpM während 2 h schloß sich an. Nach dem Dekantieren wurden die Dane-Partikel in 0,01 M Tris-Puffer (pH 7,4) aufgenommen. Als Maß für die Konzentration der Dane-Partikel erfolgte die Bestimmung der DNS-Polymerase-Aktivität nach der von KAPLAN u.a. beschriebenen Methode (vgl. J. Virol. 12 (1973), S. 995 ff.). Hierbei wird nach Zugabe aller vier Desoxyribonukleosid-triphosphate die neu synthetisierte DNS gemessen, indem die Radioaktivität im Flüssigkeitszintillationszähler bestimmt wird.
D Desinfektionsmittelversuche:
   In den nachfolgenden Versuchen diente als Desinfektionsmittel eine 2 Gew.-%ige wäßrige Zitronensäurelösung, die durch Auflösen einer im Handel erhältlichen Zitronensäure (Handelsprodukt der Fa. Merck, Art 244) in zweifach destilliertem Wasser hergestellt wurde. Der pH-Wert der 2 Gew.-%igen Lösung war 2,2.
   Die Desinfektionsmittelversuche wurden in Polyallomer-Röhrchen angesetzt und in einem Wasserbad bei 20°C und bei 75°C entsprechend der Richtlinie des Bundesgesundheitsamtes und der Deutschen Vereinigung zur Bekämpfung der Viruskrankheiten e.V. durchgeführt. Der Versuchsansatz bestand aus einem Volumenteil Dane-Partikel-Suspension, einem Volumenteil 0,01 M Tris-Puffer und 8 Volumenteilen der 2 Gew.-%igen Zitronensäurelösung als Desinfektionsmittel.
   Bei den Versuchen mit Eiweißbelastung wurde der Tris-Puffer durch eine 2 %ige Rinderserumalbumin-Lösung [bovine serum albumin, (BSA) der Fa. Beringwerke] bzw. durch foetales Kälberserum (FKS der Fa. Boehringer Mannheim) ersetzt.
   Ferner folgte ein Versuchsansatz ohne Viruszugabe, um auf diese Weise ggfls. eine Unspezifität im DNS-Polymerase-Test durch vorhandene Desinfektionsmittel zu erkennen.
   Als positive Kontrolle wurde eine 1,75 Gew.-%ige Formaldehydlösung (pH 7,0) mitgeführt. Diese Lösung hatte in früheren Versuchen eine HBV-Wirksamkeit im DNS-Polymerase-Test gezeigt.
   Unmittelbar nach Ablauf der Einwirkungszeit wurde mit 2,4 ml 0,01 M Tris-Puffer verdünnt, um durch Verdünnung die Wirkung des Desinfektionsmittels aufzuheben, wonach mit 2 ml einer 20 Gew.-%igen Saccharoselösung unterschichtet wurde. Anschließend erfolgte eine Zentrifugation in einer Zentrifuge vom Typ SW 60 Ti Rotor mit 50.000 UpM bei 4°C während 2 Stunden. Die Sedimente wurden nach Dekantieren des Überstandes und nach Trocknen im Exsikkator in 50 µl 0,01 M Tris-Puffer resuspendiert, und 25 µl wurden für die Bestimmung der DNS-Polymerase-Aktivität in diesem Test eingesetzt.
   Gemäß einem weiteren Versuch wurde eine 0,4 Gew.-%ige Zitronensäurelösung bei 60°C und ein Gemisch aus 0,6 Gew.-% Zitronensäure, 0,06 Gew.-% Milchsäure und 0,06 Gew.-% Äpfelsäure ebenfalls bei 60°C zu Desinfektionsmittelzwecken eingesetzt.
E Ergebnisse:
   Die Ergebnisse der unter Verwendung von partiell gereinigten Dane-Partikeln durchgeführten Inaktivierungsversuche mit der 2 Gew.- %igen wäßrigen Zitronensäurelösung als Desinfektionsmittel sind in den Tabellen 1 und 2, sowie die mit einer 1,75 Gew.-%igen wäßrigen Formaldehydlösung als Kontrolle durchgeführten Versuche in Tabelle 3 enthalten. Die eingesetze Dane-Partikel-Suspension war so eingestellt, daß 100 µl dieser Lösung ca. 20.000 Counts pro Minute (cpm) ergaben. Damit war sichergestellt, daß sich die HBV-Konzentration im Versuchsansatz auf = 10⁸ Dane-Partikel belief.
   Tabelle 1 zeigt die Versuchsergebnisse, die bei erfindungsgemäßem Einsatz der 2 Gew.- %igen Zitronensäurelösung als Desinfektionsmittel erhalten wurden. Die Messungen erfolgten nach einer Einwirkungszeit von 5,15 und 30 Minuten, um auch eine Aussage zur Kinetik machen zu können. Als Ausgangsdaten für die Abnahme der HBV-Aktivität dienten die Werte, die in Gegenwart von Tris-Puffer erhalten worden waren. Durch den Ansatz ohne Viruszugabe wurde ein möglicher Einfluß der Zitronensäure auf das Testsystem untersucht und damit auch auf diese Weise die untere Nachweisbarkeitsschwelle definiert. Für die 2 Gew.-%ige Zitronensäurelösung wurden die Werte 10, 10 und 12 cpm in den drei gewählten Ansätzen gefunden.
   Aus Tabelle 1 ergibt sich, daß bereits nach fünfminütiger Einwirkungszeit Counts zu finden waren, die im Bereich der Ansätze lagen, die mit Tris-Puffer und ohne Viruszugabe durchgeführt worden waren. Damit ist die untere Nachweisbarkeitsschwelle erreicht worden, und keine virale Aktivität im DNS-Polymerase-Test mehr meßbar. Diese Aussage gilt auch für die Proben, die Eiweiß im Ansatz enthielten.
   Um den Einfluß des Desinfektionsmittels und die Bedeutung der thermischen Behandlung getrennt zu studieren, wurden überdies weitere Versuche durchgeführt. Dabei wurde die Wirksamkeit der 2 Gew.-%igen Zitronensäurelösung bei 20°C, sowie der Einfluß der Temperatur ohne Desinfektionsmittelzugabe untersucht. Die entsprechenden Ergebnisse sind in Tabelle 2 enthalten.
   Aus dieser Tabelle ist zu ersehen, daß die 1,5 Gew.-%ige Zitronensäurelösung schon bei 20°C in der Lage ist, den initialen Virustiter entscheidend zu senekn. Auch hier wurden nach 5 Minuten Countzahlen gefunden, die im Bereich der Kontrollen lagen und damit den Background darstellen. Demgegenüber wurde ermittelt, daß der alleinige Einfluß der Temperatur bei 75°C nicht ausreichend ist, die virale Aktivität im DNS-Polymerase-Test vollständig zu eliminieren. Auch nach 5 und 15 Minuten Einwirkungszeit wurden Werte gefunden, die noch deutlich über den entsprechenden Daten im Zitronensäure-Ansatz lagen. Bemerkenswert erscheint, daß die höchsten Countzahlen jeweils im Ansatz mit foetalem Kälberserum zu finden waren.
   Bei den Inaktivierungsversuchen wurden zur Kontrolle eine 1,75 Gew.-%ige Formaldehydlösung mitgeführt, nachdem sich bei früheren Versuchen ergeben hatte, daß die 0,7 Gew.-%ige Lösung, die nach der Richtlinie des Bundesgesundheitsamtes und der Deutschen Vereinigung zur Bekämpfung der Viruskrankheiten e.V. (DVV) von chemischen Desinfektionsmitteln auf Wirksamkeit gegen Viren vorgesehen ist, nicht in der Lage war, den HBV-Titer innerhalb von 60 Minuten Einwirkungszeit deutlich zu reduzieren.
   Die Untersuchungsergebnisse der Formaldehyd-Vergleichsversuche sind in Tabelle 3 enthalten. Hieraus ist ersichtlich, daß die Einwirkungszeit 60 Minuten betragen muß, um die HBV-Aktivität, wie sie im DNS-Polymerase-Test gemessen wird, vollständig zu eliminieren. Nach dieser Zeit wurde eine Countzahl von 24 gefunden; diese ist ein Wert, der auch im Ansatz ohne Viruszugabe gemessen worden war.
   In Tabelle 4 sind die Versuche bei 60°C mit einer 0,4 Gew.-%igen Zitronensäurelösung bzw. mit einem Gemisch einer 06 Gew.-%igen Zitronensäure, 0,006 Gew.-% Äpfelsäure und 0,06 Gew.-% Milchsäure angegeben.

### Beispiel 3

A Testmethode:
   Die sporoziden Eigenschaften einer Desinfektionsmittelösung mit einem Gehalt in Wirkstoffen von 0,5, 1,0 bzw. 1,5 Gew.-% untersucht, wobei die Wirkstoffkombination aus 80% Zitronensäure, 10% Äfelsäure und 10% Milchsäure bestand. Untersucht wurden die sporoziden Eigenschaften bei drei Temperaturen, nämlich 73° C, 83° C und 93° C gegenüber Bac. subtilis ATCC 6051 im Wasserbad nach dem quantitativen Suspensionstest der Deutschen Gesellschaft für Hygiene und Mikrobiologie (DGHM). Auf die Richtlinien der DGHM wird hiermit ausdrücklich bezug genommen. Die Sporenzahl der Ausgangssuspension pro ml betrug 9,80 log. Einheiten. Das Inaktivierungsgemisch wies 3% Tween 80, 3% Saponin, 0,1% Histidin und 0,1% Cystein auf.
B Ergebnisse:
   Die Ergebnisse der Überprüfung der sporoziden Wirksamkeit des Desinfektionsmittels sind in der Tabelle 6 dargestellt. Man kann erkennen, daß das Desinfektionsmittel auch gegen bakterielle Sporen bei 93° C wirksam ist.

### Erläuterungen zu den Ansätzen gemäß Tabelle 1 und 2:

### Dane-Partikel-Kontrollen:

I Dane-Partikel + Aqua bidest. + Tris-Puffer
II Dane-Partikel + 2 % Albumin + Tris-Puffer
III Dane-Partikel + FKS + Tris-Puffer

### Desinfektionsmittelkontrollen:

I Tris-Puffer + Aqua bidest. + Desinfektionsmittel*
II Tris-Puffer + 2 % Albumin + Desinfektionsmittel
III Tris-Puffer + FKS + Desinfektionsmittel

* 2 %-ige wäßrige Zitronensäurelösung

### Inaktivierungsansatz:

I Dane-Partikel + Aqua bidest. + Desinfektionsmittel*
II Dane-Partikel + 2 % Albumin + Desinfektionsmittel
III Dane-Partikel + FKS + Desinfektionsmittel

### Volumenverhältnis bei allen Ansätzen I - III:

1 Volumenteil + 1 Volumenteil + 8 Volumenteile

**Tabelle 1**

| HBV-inaktivierende Eigenschaften einer 2 %-igen Zitronensäurelösung bei 75°C. Angegeben sind die Counts pro Minute (cpm). | | | | | | | | | |
|---|---|---|---|---|---|---|---|---|---|
| Einwirkzeit (Minuten) | Dane-Partikel-Kontrollen | | | Desinfektionsmittel-Kontrollen | | | Inaktivierungsansatz (2 % Zitronensäure) | | |
| | I | II | III | I | II | III | I | II | III |
| 5 | 4788 | 4666 | 4781 | n.d. | n.d. | n.d. | 11 | 8 | 9 |
| 15 | n.d. | n.d. | n.d. | n.d. | n.d. | n.d. | 12 | 9 | 7 |
| 30 | 4818 | 4517 | 4619 | 10 | 10 | 12 | 13 | 10 | 10 |
| n.d. = nicht durchgeführt | | | | | | | | | |

**Tabelle 2**

| HBV-inaktivierende Eigenschaften der 2 %igen Zitronensäurelösung bei 20°C sowie Thermostabilität des HBV bei 75°C. Angegeben sind die Counts pro Minute (cpm). | | | | | | |
|---|---|---|---|---|---|---|
| Einwirkzeit (Minuten) | Zitronensäure (2%) | | | Tris-Puffer bei 75°C | | |
| | I | II | III | I | II | III |
| 5 | 14 | 18 | 11 | 135 | 240 | 275 |
| 15 | 9 | n.d. | n.d. | 105 | 190 | 220 |
| 30 | 10 | n.d. | n.d. | n.d. | n.d. | n.d. |
| n.d. = nicht durchgeführt | | | | | | |

**Tabelle 3**

| HBV-inaktivierende Eigenschaften von Formaldehyd (Kontrolle). Angegeben sind die Counts pro Minute (cpm). | |
|---|---|
| Einwirkzeit (Minuten) | Formaldehyd (1,75%) |
| 1 | n.d. |
| 15 | 485 |
| 30 | 121 |
| 60 | 24 |
| n.d. = nicht durchgeführt | |

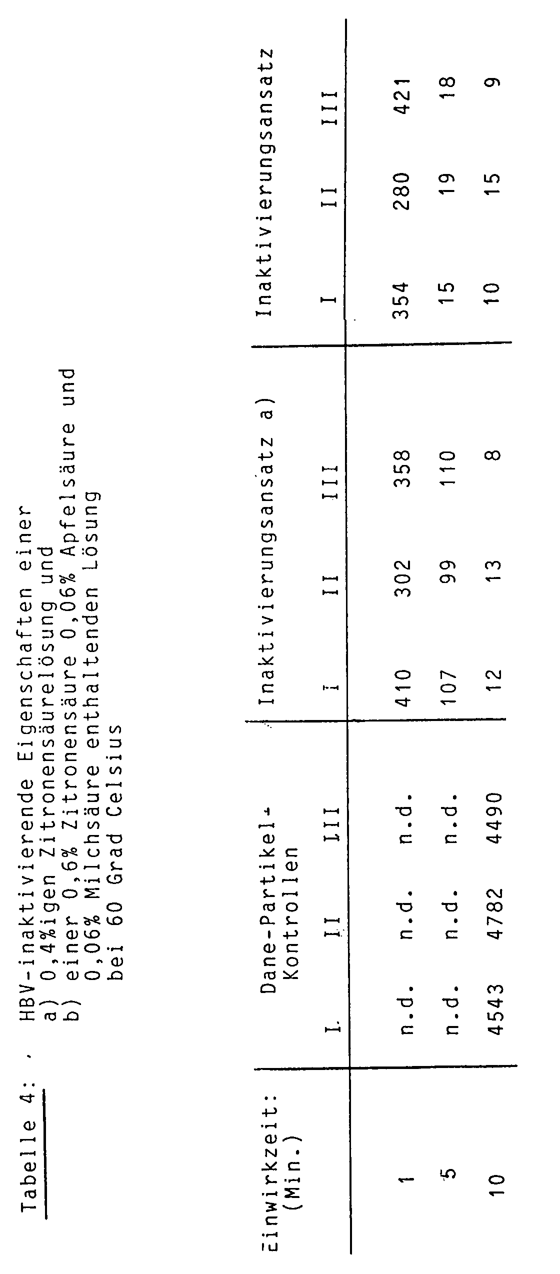

**Tabelle 6**

| Einwirkungstemperatur (%) Konzentration (%) | Reduktionsfaktoren nach Einwirkungszeit (min) | | |
|---|---|---|---|
| | 5 | 10 | 15 |
| 73 | | | |
| 1,5 | 1,50 | 1,88 | 1,99 |
| 1,0 | 0,88 | 1,85 | 1,93 |
| 0,5 | 0,72 | 0,73 | 1,16 |
| Kontrolle (WSH/73°) | 6,69 | 6,67 | 6,47 |

| 83 | | | |
|---|---|---|---|
| 1,5 | 2,39 | 3,82 | 3,85 |
| 1,0 | 2,20 | 3,34 | 3,35 |
| 0,5 | 1,80 | 2,74 | 2,86 |
| Kontrolle (WSH/83°C) | 6,40 | 6,40 | 6,27 |

| 93 | | | |
|---|---|---|---|
| 1,5 | 3,90 | 4,20 | 5,29 |
| 1,0 | 3,84 | 3,90 | 4,33 |
| 0,5 | 3,36 | 3,36 | 4,17 |
| Kontrolle (WSH/93°C) | 6,51 | 6,11 | 5,90 |
| Kontrolle (WSH/Zi-Temp) | 6,71 | 6,62 | 6,59 |
| WSH = Wasser standardisierter Härte Zi-Temp = Raumtemperatur | | | |

## Patentansprüche

1. Verwendung eines gegegenenfalls in Konzentratform vorliegenden wäßrigen Desinfektionsmittels mit einem Gehalt an Zitronensäure als viruzidem Wirkstoff zur Inaktivierung von Hepatitis-B-Viren und/oder bakteriellen Sporen bei der Desinfektion von thermolabilen medizinischen Instrumenten und Geräten sowie Teilen derselben, Dialysemaschinen und Flächen.

2. Verwendung von Milchsäure, Äpfelsäure und/oder Weinsäure als zusätzlichen Wirkstoff nach Anspruch 1.

3. Verwendung von Zitronensäure in einer Konzentration von 0,05-3 Gew.-%, insbesondere 0,4-0,6 Gew.-% nach Anspruch 1.

4. Verwendung nach Anspruch 2, dadurch gekennzeichnet, daß der zusätzliche Wirkstof jeweils in einer Konzentration von 5-20 Gew.-%, vorzugsweise etwa 10 Gew.-%, bezogen auf den Zitronensäuregehalt, vorliegt.

5. Verwendung nach Anspruch 1, dadurch gekennzeichnet, daß das Desinfektionsmittel aus einer 50 Gew.-%igen Zitronensäurelösung bestehend als Konzentrat vorliegt.

6. Verwendung nach Anspruch 1, gekennzeichnet durch einen Gehalt an 21 Gew.-% Zitronensäure, 2 Gew.-% Äpfelsäure und 2 Gew.-% Milchsäure.

7. Verfahren zum Desinfizieren von mit Hepatoviren, insbesondere Hepatitis-B-Viren, kontaminierten Flächen, insbesondere thermolabiler medizinischer Instrumente und Geräte sowie Teilen derselben, dadurch gekennzeichnet, daß man das Desinfektionsmittel gemäß einem der Ansprüche 1 bis 4 bei einer Temperatur von 20°C bis 75°C während zumindest 5 Minuten in Berührung bringt.

8. Verfahren gemäß Anspruch 7, dadurch gekennzeichnet, daß man das Desinfektionsmittel gemäß Ansprüchen 2 und 4 bei 20°C während 5 Minuten mit den Flächen in Berührung bringt.

9. Verwendung eines Desinfektionsmittels nach einem der Ansprüche 1 bis 7 zur Inaktivierung von Legionella pneumophila bei der Desinfektion von insbesondere thermolabilen medizinischen Instrumenten und Geräten sowie Teilen derselben, Dialysemaschinen und Flächen.

## Claims

1. The use of an aqueous disinfection material, if necessary in form of a concentrate, having a concentration of citric acid as virucidic component for deactivation of hepatitis-B-viruses and/or bacterial spores with the disinfection of temperature sensitive medicinal instruments and apparatuses as well as parts thereof, dialysis machines and surfaces.

2. The use of lactid acid, malic acid and/or tartaric acid as additional component according to Claim 1.

3. The use of citric acid in a concentration from 0,05-3 wt. %, in particular 0,4-0,6 wt. % according to Claim 1.

4. The use according to Claim 2, characterized in that of the additional component is present in a concentration of 5-20 wt. %, preferably about 10 wt. %, referred to the citric acid content.

5. The use according to Claim 1, characterized in that the disinfection material is present as concentrate consisting of a 50 wt. % citric acid solution.

6. The use according to Claim 1, characterized by a content of 21 % citric acid, 2 % malic acid and 2 % lactic acid, all by weight.

7. A process for the disinfection of surfaces contaminated with hepato viruses, in particular hepatitis-B-viruses, of in particular temperature sensitive medicinal instruments and apparatuses as well as parts thereof, characterized in that the disinfection material according to any of Claims 1 to 4 is brought into contact at a temperature of 20°C to 75°C for at least 5 minutes.

8. The process according to Claim 7, characterized in that the disinfection material according to Claims 2 and 4 is brought into contact with the surfaces at 20°C for 5 minutes.

9. Use of a disinfection material according to any of Claims 1-7 for deactivation of legionella pneumophilia with the disinfection of in particular temperature sensitive medicinal instruments and apparatuses as well as parts thereof, dialysis machines and surfaces.

## Revendications

1. Utilisation d'un désinfectant aqueux éventuellement présent sous forme d'un concentrat, possédant une teneur en acide citrique comme ingrédient actif virucide pour l'inactivation de virus de l'hépatite B et/ou de spores bactériennes lors de la désinfection d'instruments et d'appareils médicaux thermolabiles, ainsi que de parties de ces derniers, d'appareils de dialyse et de surfaces.

2. Utilisation d'acide lactique, d'acide malique et/ou d'acide tartrique comme ingrédient actif supplémentaire selon la revendication 1.

3. Utilisation d'acide citrique en une concentration de 0,05-3% en poids, en particulier de 0,4-0,6% en poids, selon la revendication 1.

4. Utilisation selon la revendication 2, caractérisée en ce que l'ingrédient actif supplémentaire est présent respectivement en une concentration de 5-20% en poids, de préférence d'environ 10% en poids rapportés à la teneur en acide citrique.

5. Utilisation selon la revendication 1, caractérisée en ce que le désinfectant constitué par une solution d'acide citrique à 50% en poids est présent sous forme d'un concentrat.

6. Utilisation selon la revendication 1, caractérisée par une teneur de 21% en poids d'acide citrique, de 10% en poids d'acide malique et de 2% en poids d'acide lactique.

7. Procédé pour désinfecter des surfaces contaminées avec des hépatovirus, en particulier des virus de l'hépatite B, d'instruments et d'appareils médicaux en particulier thermolabiles, ainsi que des parties de ces derniers, caractérisé en ce qu'on amène le désinfectant, selon l'une quelconque des revendications 1 à 4, à une température de 20°C à 75°C, en contact pendant au moins 5 minutes.

8. Procédé selon la revendication 7, caractérisé en ce qu'on amène le désinfectant, selon les revendications 2 et 4, à une température de 20°C pendant 5 minutes, en contact avec les surfaces.

9. Utilisation d'un désinfectant selon l'une quelconque des revendications 1 à 7, pour l'inactivation de Legionella pneumophila lors de la désinfection d'instruments et d'appareils médicaux en particulier thermolabiles, ainsi que de parties de ces derniers, d'appareils de dialyse et de surfaces.
